Europäisches Patentamt

(19) European Patent Office

Office européen des brevets

(11) Publication number : **0 414 080 B1**

(12) **EUROPEAN PATENT SPECIFICATION**

(45) Date of publication of patent specification :
16.02.94 Bulletin 94/07

(51) Int. Cl.⁵ : **A61K 37/26, A61K 9/48, A61K 9/70, A61K 47/12**

(21) Application number : 90115452.6

(22) Date of filing : 11.08.90

(54) **Non-parenteral pharmaceutical compositions for insulin.**

(30) Priority : 21.08.89 US 396486

(43) Date of publication of application :
27.02.91 Bulletin 91/09

(45) Publication of the grant of the patent :
16.02.94 Bulletin 94/07

(84) Designated Contracting States :
AT BE CH DE DK ES FR GB IT LI LU NL SE

(56) References cited :
EP-A- 0 225 189
EP-A- 0 351 651
WO-A-87/06137
STN INFORMATION SERVICES DATA BASE:
CHEMICAL ABSTRACTS, vol. 110, no. 16, 1989,
Columbus, OH (US); M. MISHIMA et al., no.
141429y

(73) Proprietor : F. HOFFMANN-LA ROCHE AG
Postfach 3255
CH-4002 Basel (CH)

(72) Inventor : Campfield, Leroy Arthur
35 Depot Street
Verona, N.J. 07044 (US)
Inventor : Davidovich, Alberto
29 Kimberly Place
Wayne, N.J. 07470 (US)
Inventor : Infeld, Martin Howard
6 Tuers Place
Upper Montclair, N.J. 07043 (US)
Inventor : Shah, Navnit
203 Beverly Hill Road
Clifton, N.J. 07012 (US)
Inventor : Smith, Françoise Jeanne
55 Depot Street
Verona N.J. 07044 (US)
Inventor : Unowsky, Joel
203 East McClellan Avenue
Livingston, N.J. 07039 (US)

(74) Representative : Grossner, Lutz, Dr. et al
Grenzacher Strasse 124 Postfach 3255
CH-4002 Basel (CH)

## Description

Briefly described, this invention is based on the discovery that alkali metal salts of coconut oil are highly effective in promoting the absorption and bioavailability of insulin via routes of administration other than parenteral injection. Such a substance can be formulated together with insulin into various pharmaceutical dosage forms which are useful for treating conditions of hyperglycemia, such as diabetes mellitus. Suitable dosage forms comprise oral, rectal, buccal, sublingual and intranasal.

In greater detail, the present invention relates to non-parenteral pharmaceutical compositions comprising

(a) a blood sugar-lowering amount of insulin,

(b) an amount of an alkali metal cocoate sufficient to enhance the absorption of the insulin into the body, and

(c) optionally, a pharmaceutically acceptable carrier.

The absorption enhancing agent, component (b), is a derivative of the naturally occurring substance coconut oil. Typically, coconut oil is composed of a mixture of straight chain, saturated $C_8$-$C_{18}$ fatty acids, e.g., caprylic, capric, lauric, myristic, palmitic and stearic, as well as relatively small amounts of unsaturated $C_{18}$-$C_{20}$ fatty acids, or esters of such saturated and unsaturated fatty acids with glycerol.

Suitable alkali metals for salt formation with the coconut oil (or oils) are sodium, potassium, etc., with sodium and potassium being preferred.

Procedures for the preparation of such salts are known. For instance, in one procedure, coconut oil is reacted with an alkali metal hydroxide (e.g., sodium hydroxide, potassium hydroxide, etc.), and the reaction mixture is autoclaved, with glycerin being formed as a byproduct.

A suitable absorption enhancing agent for use in this invention which is commercially available is NORFOX 1101, a potassium cocoate, produced by Norman Fox and Company, Vernon, California.

In providing the various dosage form compositions in accordance with this invention, the described absorption enhancing agent, component (b), is utilizable with all types of insulin effective for lowering blood sugar in the body, including but not limited to animal-source products such as crystalline forms derived from cattle (bovine) or swine (porcine) pancreatic glands (e.g., Eli Lilly's Iletin® insulins), as well as recombinant DNA-originated forms of human insulin (e.g., Lilly's Humulin®).

In addition to these more traditional types of mammalian insulin, the invention is also useful with other forms of insulin suitable for administration to humans, including various salts, derivatives and conjugates for modifying its duration of activity or other properties. Also encompassed are chemically modified derivatives of naturally occurring insulins, for example, newer molecules in which one or more amino acids have been omitted or substituted to change the properties.

In general, individual dosage forms in accordance with this invention can be composed to contain the insulin in amounts typical for the treatment of hyperglycemia, for instance, in amounts of 20, 40, 100, 200 and 500 units per dosage unit.

It is also understood that up to 1000 units per dosage form may be used.

Typically, the pharmaceutical compositions of this invention contain the absorption enhancer agent, component (b), to the insulin, component (a), in a weight ratio within the range from 500:1 to 1:1 and preferably from 50:1 to 5:1. A range of 8:1 is most preferred. For purposes of this description, each 25 units of insulin is equivalent to approximately 1 milligram of insulin.

The ratio of absorption enhancer agent, component (b), to polymer material used to form the buccal film is in a weight ratio within the range from 1:20 to 20:1, with a preferred range from 1:5 to 5:1 and most preferred 1:1.

Presently, buccal film administration is the preferred route of administration for this invention. To produce such a buccal film, for example, crystalline insulin is dissolved in an absorption enhancer to form a solution. A polymeric material, such as hydroxypropyl cellulose, hydroxypropyl methylcellulose, cellulose acetate, sodium carboxymethylcellulose, cellulose acetate butyrate, methyl methacrylate polymers, polyvinyl alcohol, polyvinylpyrrolidone or another polymer known in the art, is added to the mixture, dry or in solution. The resulting solution is cast into a film. Casting is accomplished by pouring the solution onto a nonadhesive surface and spreading it into a uniform thickness with a spreading tool, such as a doctor blade, and then drying the solution to form a film.

Hydroxypropyl cellulose and hydroxypropyl methylcellulose, which are preferred polymeric materials, have a viscosity of a 2% solution (w/v) in the range of 5 centipoise to 50,000 centipoise.

The film is dried at or about room temperature and preferably not above a temperature which would denature the insulin molecule, such as for example 37°C. Warm air may be blown over the film to dry it. The film is then cut to a specified size, determined by a desired film surface area to provide the desired dosage strength.

In addition to the insulin, the absorption enhancer and the polymeric material described above, other substances may be added to the film such as plasticizers, stabilizers, antioxidants, preservatives, colorants, buffering agents, non-caloric sweetening agents, flavoring agents and other conventional pharmaceutical additives. Plasticizers, such as polyethylene glycol and propylene glycol, may be used to obtain a desired elasticity of the film.

Bioadhesive polymers may also be added to the film to enhance the adhesion of the film to the mucosal lining of the buccal cavity. Such polymers include polycarbophil, carbopol, and other such bioadhesive polymers known in the art. The use of high temperatures, strong solvents or other severe conditions tending to denature the insulin should be avoided.

Examples of insulin formulations in accordance with this invention which can be prepared for delivery as a buccal film are as follows:

### BUCCAL FILM FORMULATIONS

| | per buccal film | per buccal film | per buccal film | per buccal film | per buccal film |
|---|---|---|---|---|---|
| **1. Insulin (crystalline)** | 60 units | 60 units | | | |
| Absorption enhancer | 40 mg | 100 mg | | | |
| Polymer material | 50 mg | 100 mg | | | |
| **2. Insulin (crystalline)** | 120 units | 120 units | 120 units | 480 units | |
| Absorption enhancer | 40 mg | 100 mg | 100 mg | 40 mg | |
| Polymer material | 50 mg | 100 mg | 50 mg | 50 mg | |
| **3. Insulin (crystalline)** | 60 units | 120 units | 120 units | 240 units | 480 units |
| Absorption enhancer | 40 mg | 40 mg | 100 mg | 40 mg | 40 mg |
| Polymer material | 50 mg | 50 mg | 100 mg | 50 mg | 50 mg |
| Polyethylene glycol | 1 mg | 1 mg | 1 mg | 1 mg | 1 mg |
| **4. Insulin (crystalline)** | 60 units | 120 units | 120 units | 240 units | 480 units |
| Absorption enhancer | 40 mg | 40 mg | 100 mg | 40 mg | 40 mg |
| Polymer material | 50 mg | 50 mg | 100 mg | 50 mg | 50 mg |
| Flavor | 5 mg | 5 mg | 5 mg | 5 mg | 5 mg |
| Sweetener | 1 mg | 1 mg | 1 mg | 1 mg | 1 mg |

In a preferred film embodiment, potassium cocoate as the absorption enhancer is dissolved in hydroxypropyl cellulose as the polymer material. An example of a hydroxypropyl cellulose is sold by Aqualon Co. of Wilmington, Delaware under the trademark Klucel[R].

The buccal film may be of any desired size and shape as is known in the art to deliver a preferred dosage of the insulin-absorption enhancer formulation and to fit within the buccal cavity either on the mucosal surface of the cheek or between the cheek and gum surfaces. Factors such as film thickness, surface area and dosage strength should be considered in the film design. One or a number of films may be used for each dosage administration, if desired. Film thickness should preferably be about 10 microns to about 200 microns and especially preferred thicknesses are about 50 microns to about 75 microns. It has been observed that a circular film of about 8-16 cm$^2$ surface area is particularly useful in delivering the inventive formulations.

It may also be appreciated that the resulting buccal film may be prepared with a removable backing to protect the bio-adhesive polymer during storage and handling.

The following examples illustrate the buccal film of the present invention:

EXAMPLE A

At about room temperature, 120 Units of crystalline porcine insulin (5 mg) was dissolved in 100 mg of potassium cocoate to form a solution. To the potassium cocoate-insulin solution, 100 mg of hydroxypropyl cellulose (Klucel LF ®) was added as a 10% solution (w/v). The insulin-potassium cocoatehydroxypropyl cellulose solution was cast into a film using a film spreading tool, such as a Gardner® Knife and the film was dried at room temperature (i.e. about 23°C). The resulting film was cut to form a circular film of about 8-16 cm$^2$ surface area and about 100 microns in thickness.

EXAMPLE B

At about room temperature, 120 Units of crystalline porcine insulin (5 mg) was dissolved in 40 mg of potassium cocoate to form a solution. To the potassium cocoate-insulin solution, 50 mg of hydroxypropyl cellulose (Klucel LF ®) was added as a 10% solution (w/v). The insulin-potassium cocoate-hydroxypropyl cellulose solution was cast into a film using a film spreading tool, such as a Gardner® Knife and the film was dried at room temperature (i.e. about 23°C). The resulting film was cut to form a circular film of about 8-16 cm$^2$ surface area and about 50 microns in thickness.

EXAMPLE C

At about room temperature, 60 Units of crystalline porcine insulin (2.5 mg) was dissolved in 100 mg of potassium cocoate to form a solution. To the potassium cocoate-insulin solution, 100 mg of hydroxypropyl cellulose (Klucel LF®) was added as a 10% solution (w/v). The insulin-potassium cocoate-hydroxypropyl cellulose solution was cast into a film using a film spreading tool, such as a Gardner® Knife and the film was dried at room temperature (i.e. about 23°C). The resulting film was cut to form a circular film of about 8-16 cm$^2$ surface area and about 50 microns in thickness.

ORAL ADMINISTRATION

Another preferred route of administration for this invention is oral administration. For oral administration, the composition can be administered in the form of soft shell capsules, hard shell capsules, microcapsules, microspheres or tablets, optionally in admixture with talc or other inert ingredients, that is, pharmaceutically acceptable carriers, or in the form of aqueous solutions or suspensions, for example, in admixture with non-caloric sweetening agents, flavoring agents, stabilizers, antioxidants, preservatives, buffering agents, lubricants, colorants, thickeners and other conventional pharmaceutical additives. The capsules, spheres, tablets, solutions, etc. can be prepared in accordance with conventional methods known for these purposes.

The use of capsules is especially preferred. By way of illustration, such dosage forms are conveniently prepared by dissolving or dispersing at room temperature, with gentle mixing, a measured amount of insulin in an appropriate amount of the absorption enhancing agent, then loading into a suitable hard- or soft shell capsule (e.g., gelatin). Alternatively, if water is used, the insulin is first dissolved or suspended in water at room temperature with gentle mixing, and the insulin-water solution or suspension is added slowly to the absorption enhancing agent with mixing continued to form a uniform three-component mixture. It may be necessary to add a buffer or buffering agent to adjust the pH to within a range in which the insulin is both soluble and stable. Care should be taken not to use so much water as to dissolve or otherwise cause physical deterioration of the capsule, as will be understood by the skilled practitioner. The use of strong solvents, high temperatures or other severe conditions tending to denature the insulin should be avoided.

Examples of insulin formulations in accordance with this invention which can be prepared by the above mentioned procedures for subsequent loading into capsules are as follows:

## ORAL DOSAGE FORMULATIONS

|    |                       | per capsule | per capsule | per capsule |
|----|-----------------------|-------------|-------------|-------------|
| 1) | Insulin (crystalline) | 120 units   | 240 units   | 480 units   |
|    | Absorption enhancer   | 50 mg       | 100 mg      | 100 mg      |
| 2) | Insulin (crystalline) | 120 units   | 240 units   | 480 units   |
|    | Absorption enhancer   | 100 mg      | 200 mg      | 400 mg      |

Preferably, the resulting capsules are provided with an enteric coating which is resistant to gastric fluids and is unaffected by them but which dissolves readily in the intestinal fluid, thereby causing insulin release.

The efficacy of particular enteric coating materials considered for use in the practice of this invention can be measured using known USP methods. By way of illustration, suitable enteric coating materials for purposes of the invention include but are not limited to the following:

### Enteric Coating Formulations

| Ingredients | % w/w |
|-------------|-------|

**Solution A:**

| | |
|---|---|
| Hydroxypropyl methylcellulose phthalate (HPMCP) | 5.0 |
| Triacetin | 0.5 |
| Methylene chloride | 47.25 |
| Denatured alcohol | 47.25 |

**Solution B:**

| | |
|---|---|
| HPMCP | 10.0 |
| Titanium dioxide | 0.2 |
| Triethyl citrate | 0.05 |
| Acetone | 44.875 |
| Denatured alcohol | 44.875 |

Solution C:

| | |
|---|---|
| Cellulose acetate phthalate (CAP) | 8.5 |
| Diethyl phthalate | 1.5 |
| Titanium dioxide | 0.2 |
| Acetone | 44.9 |
| Denatured alcohol | 44.9 |

Solution D:

| | |
|---|---|
| Polyvinyl acetate phthalate | 5.0 |
| Acetylated glycerides | 0.8 |
| Methylene chloride | 47.1 |
| Denatured alcohol | 47.1 |

Solution E:

| | |
|---|---|
| Methacrylic acid or methacrylic acid ester (Eudragit S or L, Rohm Pharma, GMBH, Wetterstadt, West Germany) | 8.0 |
| Acetone | 45.5 |
| Anhydrous alcohol | 45.5 |
| Plasticizer | 1.0. |

These enteric coating materials may be applied with or without plasticizers, for example, acetylated monoglycerides, dibutyl phthalate, triethyl citrate, diethylphthalate, triacetin and dibutyl sebacate, using methods known to those skilled in the art.

The percentage of enteric coating applied is usually between about 1 and about 20 percent by weight, or more, and most desirably from about 2 to about 8 percent by weight, based on the total capsule weight.

For rectal administration, the described insulin compositions can be prepared into dosage forms suitable for the purpose, e.g., capsules or suppositories. Capsules can be prepared in a manner similar to what has been described above for the oral dosage form, but without the need for an enteric coating. Suppositories can be prepared by melting a suitable suppository base, mixing the insulin, absorption enhancer and any other ingredients into the melt, pouring the molten mixture into molds, allowing to solidify, sealing the molds and storing in well-closed containers until use. Suitable suppository bases include but are not limited to those base substances which are mixtures of mono-, di- and triglycerides of $C_{12}$ to $C_{18}$ fatty acids and are available from Dynamit Nobel Company under the trade designation "WITEPSOL". Some examples of rectal formulations are given below.

## RECTAL DOSAGE FORMULATIONS

|  | per suppository | per suppository |
|---|---|---|
| Insulin (crystalline) | 120 units | 480 units |
| Absorption enhancer | 100 mg | 400 mg |
| Carrier | 780 mg | 1120 mg |

For the other types of non-parenteral administration contemplated for use with this invention, suitable dosage forms can be prepared utilizing appropriate techniques well known to those skilled in the art. Such dosage forms for buccal and sublingual administration include tablets, capsules, gels, films, patches and solutions, as well as aerosol sprays and drops for intranasal delivery. Some examples of formulations for buccal, sublingual and intranasal dosage forms are given below.

## BUCCAL/SUBLINGUAL DOSAGE FORMULATIONS

|  |  | per 0.3 ml sol. | per 0.3 ml sol. | per 0.3 ml sol. |
|---|---|---|---|---|
| 1) | Insulin (crystalline) | 120 units | 240 units | 480 units |
|  | Absorption enhancer | 50 mg | 100 mg | 100 mg |
|  | Water q.s. | 0.3 ml | 0.3 ml | 0.3 ml |
| 2) | Insulin (crystalline) | 120 units | 240 units | 480 units |
|  | Absorption enhancer | 100 mg | 100 mg | 100 mg |
|  | Orange flavor | 5 mg | 10 mg | 10 mg |
|  | Aspartame | 1 mg | 2 mg | 2 mg |
|  | Water q.s. | 0.3 ml | 0.3 ml | 0.3 ml |

|  |  | per 0.3 ml solution | per 0.6 ml solution |
|---|---|---|---|
| 3) | Insulin (crystalline) | 120 units | 480 units |
|  | Absorption enhancer | 100 mg | 200 mg |
|  | Aspartame | 1 mg | 2 mg |
|  | Orange flavor | 5 mg | 10 mg |
|  | Water q.s. | 0.3 ml | 0.6 ml |

7

## INTRANASAL DOSAGE FORMULATIONS

|  | per 0.15 ml solution | per 0.3 ml solution |
|---|---|---|
| Insulin (crystalline) | 120 units | 480 units |
| Absorption enhancer | 50 mg | 100 mg |
| Water q.s. | 0.15 ml | 0.3 ml |

Any of the just aforementioned dosage form compositions in accordance with this invention can also be formulated to contain pharmaceutically acceptable adjuvants typical for the particular dosage form involved, including but not limited to stabilizers, flavoring agents, antioxidants, preservatives, buffering agents, colorants, bulking agents, lubricants, and so forth, which may be selected from among materials known for such purposes and added in conventional amounts.

The usefulness of compositions according to this invention for the treatment of hyperglycemia is demonstrated in two separate in vivo animal models (rats and dogs), as follows:

### IN VIVO (RATS)

Female Wistar rats and Sprague-Dawley rats, having a body weight of from 210 to 260 grams, were deprived of food for 1 to 2 hours prior to surgery. The animals were anesthetized with sodium pentobarbital, using 50 mg of the anesthetic per kilogram of animal body weight, and cardiac and duodenal cannulas were then implanted. Before surgery, the cardiac cannulas were filled and mounted with 1 c.c. syringes containing heparin saline solution at a concentration of 50 units per milliliter. The cardiac cannulas were implanted in the rats through the jugular vein at or about 34 mm toward the atria (For additional details on the procedure, see Smith and Campfield, American Journal of Physiology, 251: R70-76, 1986).

The duodenal cannulas were made of 0.86 mm (inner diameter) polyethylene tubing having a length of 150 mm and mounted with a 1 c.c. syringe filled with 0.9% saline solution. Implantation of the duodenal cannulas was made through an incision in the duodenal wall 10 mm down from the pyloric valve. In each case, approximately 15 mm of the tubing was inserted through the incision and tied with sutures around the incision.

All incisions were closed with sutures. Following completion of the surgery, 200 units of heparin (0.2 ml of 1000 units/ml, followed by 0.2 ml of isotonic saline) were injected into the cardiac cannula. The experimental protocol was commenced 60 minutes later.

### Insulin Administration

Insulin (Eli Lilly's Iletin® U500, 500 units per milliliter) was gently mixed with varying concentrations of potassium cocoate prior to infusion. Particular concentrations of these materials in each incidence of administration are detailed further below and shown in the accompanying graphs. For each test animal, a 0.5 ml dose of solution was infused into the exteriorized portion of the implanted duodenal cannula, using a 1 c.c. tuberculin syringe attached to a 20-gauge needle.

### Continuous Blood Glucose Recording

A YSI Model 23A Glucose Analyzer (Yellow Springs Instrument Co., Inc.) and glucose-oxidase method were used to continuously measure the blood glucose concentration, in units of mg/dl, in rats. (For details, see Campfield et al., Brain Research Bulletin 14: 605-616 (1985)).

Following at least 10 minutes of stable blood glucose monitoring ($\pm$ 1%), administration of insulin-absorption enhancer mixture was begun and blood glucose recording was continued for up to 60 minutes. Upon termination of experiments in each test rat, the correct placement of the duodenal cannula was verified by administration of 1 ml of trypan blue. This allowed verification of proper intestinal delivery of the mixture. Changes in the level of blood glucose were calculated as a function of time and magnitude over the baseline. At least three runs were performed, each on a different test rat. If the infusion of insulin and absorption enhancer significantly reduced the blood glucose level (i.e., greater than 10%), a dose-response curve of the insulin-absorption enhancer mixture was further evaluated in discrete sampling experiments, the procedure of which is described below.

## Discrete Sampling

Baseline blood samples of 0.25 ml volume were collected from the test rat at 15 minutes prior to administration of the insulin-absorption enhancer mixture. Blood samples were also collected at 2, 15, 20, 30, 45 and 60 minute-intervals following insulin-absorption enhancer administration. Blood volume was replaced after withdrawal of each sample with 0.25 ml of isotonic saline. Withdrawn blood samples were immediately spun using a micro-centrifuge (Eppendorf Co., Model No. 5415) for 2 minutes at 14,000 rpm. Plasma was collected into micro-beakers and stored at -20°C for subsequent determination of glucose and insulin concentrations, using the assay procedures described below.

## Plasma Glucose and Insulin Assays

The plasma glucose concentrations of the test rats over time were measured in 25-microliter samples using a YSI Model 23A Glucose Analyzer, Yellow Springs Instrument Co., Inc., which employs a glucose-oxidase method (see reference above for details). The results were expressed in mg/dl and percent change of baseline. Data were averaged across test groups and expressed as mean $\pm$ S.E.M.

The plasma insulin concentrations of the test rats were measured in 25-microliter samples using a competitive protein binding microassay, with pork insulin as the standard (see Campfield et al., American Journal of Physiology, 244: R629-634, 1983). The minimum detectable insulin concentration was 0.04 ng/ml and the maximum readable concentration (undiluted) was 10 ng/ml. The results were expressed in ng/ml per unit time, as well as for cumulative insulin release per 60 minutes (i.e., insulin area under the curve: Trapezoidal Rule Algorithm). Data were averaged across test groups and expressed as mean $\pm$ S.E.M.

## Continuous Blood Glucose Recording

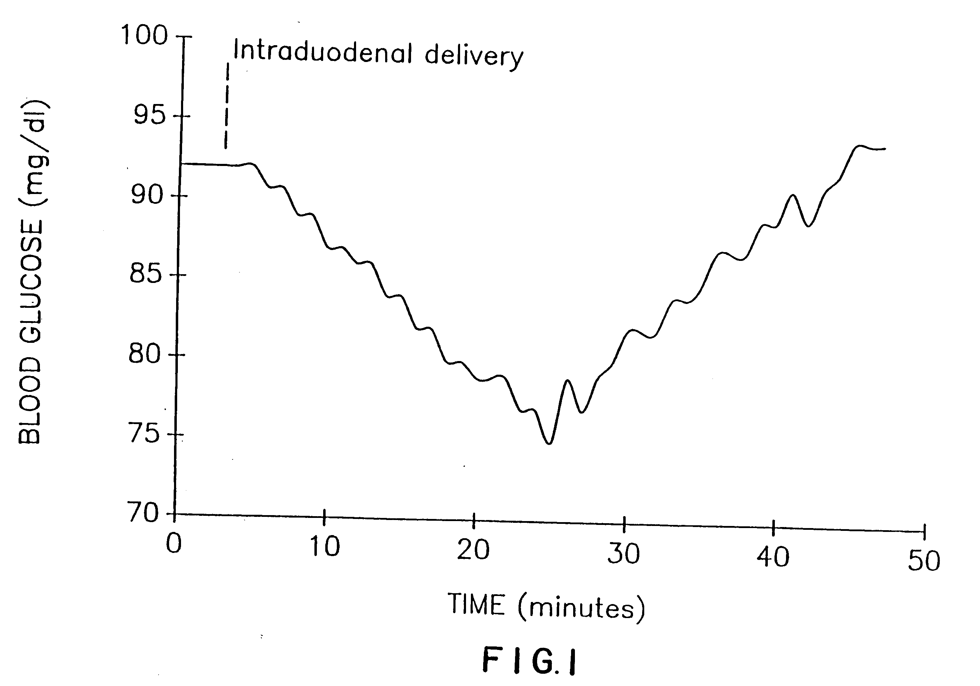

Three anesthetized rats fitted with intraduodenal cannulas received one acute dose of 1.84 mg of potassium cocoate and 20 units of porcine insulin. The effect of this infusion on the blood glucose concentration as a function of time is shown in Fig. 1.

Blood glucose decreased to 18.5% below baseline concentration at 20 minutes after the infusion. Blood glucose returned to the baseline at 39 minutes.

## Dose-Response Studies of the Absorption of 20 Units of Insulin as a Function of Absorption Enhancer Concentration - Single Dose (Rats)

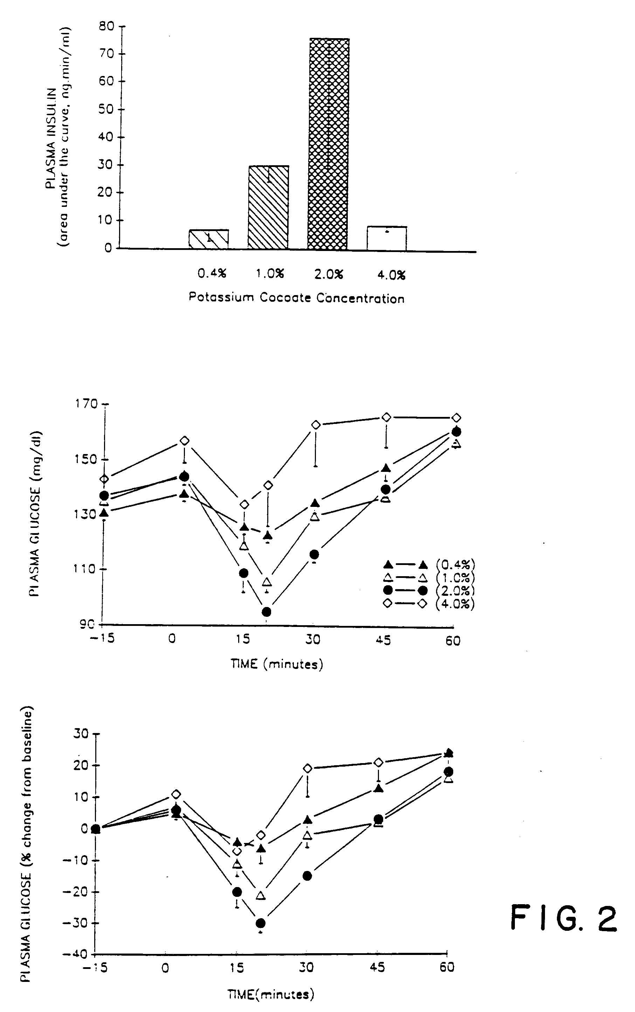

Four different concentrations of potassium cocoate as the absorption enhancer were mixed with a constant dose of 20 units of porcine insulin (Lilly's Iletin® U500) and infused intraduodenally into anesthetized rats using the method referred to above, such that the rats received 1.84, 4.6, 9.2 or 18.4 mg of the absorption enhancer, which corresponds to approximately a 0.4, 1, 2 or 4% solution, respectively, of absorption enhancer. The effect of these infusions on the plasma glucose concentration, expressed both as an absolute and a percent change from the baseline as a function of time, is shown in Figure 2. The data points represent mean $\pm$ S.E.M. Plasma insulin is expressed as the area under the curve.

As is shown, intraduodenal administration of mixtures of the absorption enhancer and insulin resulted in a decrease in the plasma glucose of about 6 to 31% after 20 minutes. The plasma glucose returned to the baseline after 45 minutes in the 4.6 and 9.2 mg groups, while it returned to the baseline after 30 minutes in the 1.84 and 18.4 mg groups. Plasma insulin rose from 6.9 to 75.9 ng.min/ml as a function of increasing concentration of the absorption enhancer (1.84 to 9.2 mg) while declining to 8.7 ng.min/ml in the 18.4 mg group.

## Dose-Response Studies of the Absorption of Insulin in the Presence of 9.2 mg of Absorption Enhancer as a Function of Insulin Concentration - Single Dose (Rats)

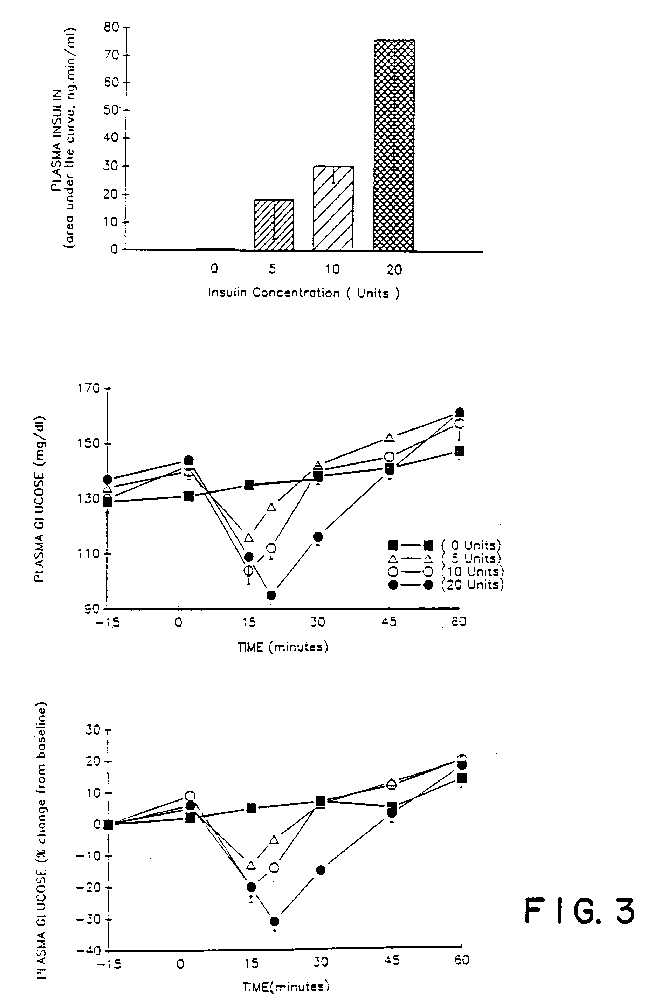

Four different doses of porcine insulin (Lilly's Iletin® U500), 0, 5, 10 or 20 units, were mixed with a constant dose of 9.2 mg (2% solution) absorption enhancer (potassium cocoate) and the mixtures were infused intraduodenally into anesthetized rats using the method referred to above. The effects on plasma glucose and insulin levels over time are shown in Figure 3. The data points represent mean $\pm$ S.E.M.

Intraduodenal administration of the absorption enhancer-insulin mixture at these doses resulted in a decrease in plasma glucose of about 13 to 31%, 15 minutes after infusion, as shown. The plasma glucose returned to the baseline level after 30 minutes in the 5 and 10 unit groups, while it returned to the baseline after 45 minutes in the 20 unit group. Administration of the absorption enhancer alone was without effect on the plasma glucose level. The plasma insulin increased from 0.68 to 75.9 ng.min/ml as a function of the insulin concentration.

Dose-Response Studies of the Absorption of Insulin in the Presence of 4.6 mg of Absorption Enhancer as a Function of Insulin Concentration - Single Dose (Rats)

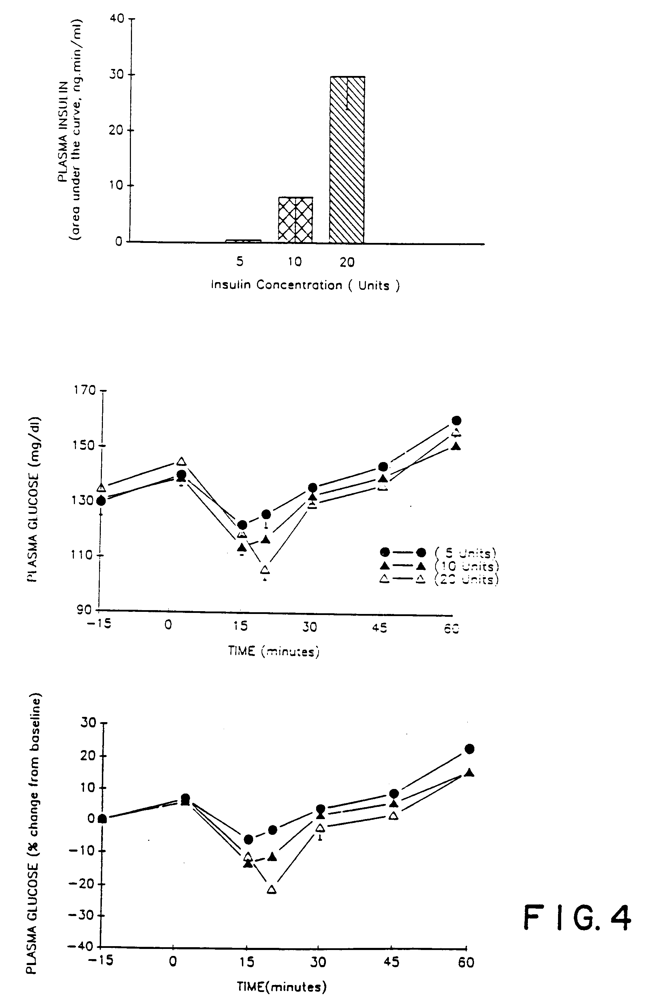

Three different doses of porcine insulin (Lilly's Iletin® U500), 5, 10 or 20 units, were infused intraduodenally into anesthetized rats using the method referred to above. The effects are shown in Figure 4, with the data points given in mean ± S.E.M.

Intraduodenal administration of absorption enhancer and insulin at these doses also resulted in a dose-dependent decrease in the plasma glucose of about 6 to 21% 20 minutes after infusion. Plasma insulin increased from 0.50 to 29.9 ng.min/ml as a function of the insulin concentration. These data demonstrate a dose-dependent absorption of insulin in the presence of 4.6 mg of the absorption enhancer.

Multiple Dose Insulin/Absorption Enhancer (Rats)

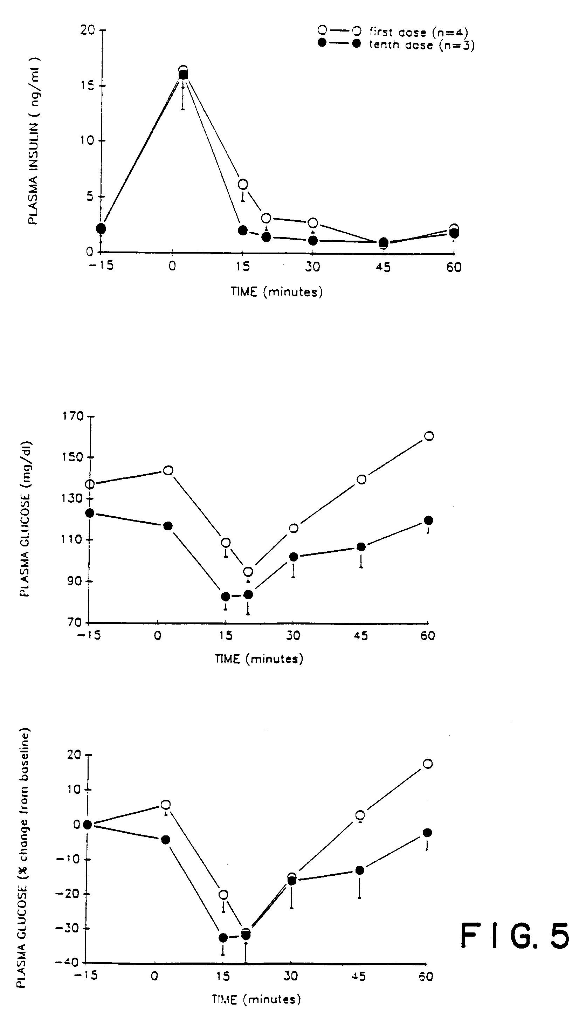

Three rats with chronic intraduodenal cannulas received nine daily doses of 9.2 mg of potassium cocoate and 20 units of porcine insulin. On the next day, the rats were anesthetized and the plasma glucose and insulin responses to the tenth dose were measured. The glucose and insulin responses following the tenth dose are compared with the responses to a single dose in naive rats of the same age and sex in Figure 5. Data are mean ± S.E.M.

Plasma glucose concentrations decreased to a nadir (32%) after 20 minutes and returned to baseline levels at 60 and 45 minutes in both multiple dose-treated and naive rats, respectively. Plasma insulin concentration rose rapidly to a large peak (8-fold) at 2 minutes post-infusion and returned to baseline at 20 minutes in both multiple dose treatment and single dose treatment groups. These studies demonstrate that the ability of 9.2 mg of potassium cocoate to enhance the intestinal absorption of insulin is retained for at least 10 days in the animals tested.

Single Dose Insulin/Absorption Enhancer (Hyperglycemic Rats)

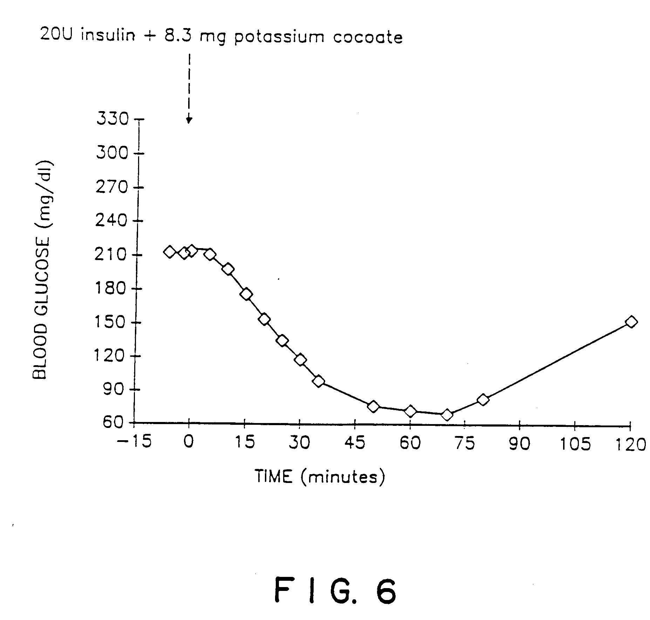

Rats were injected subcutaneously with streptozotocin (Sigma) at a dose of 45 mg/kg b.w. Water consumption increased and glycosuria was present. Twelve days after the injection, the rats were anesthetized and blood glucose was monitored following a single intraduodenal administration of 8.3 mg of potassium cocoate and 20 units of porcine insulin. The effect of the insulin-absorption enhancer mixture on the blood glucose concentration as a function of time is shown in Fig. 6.

IN VIVO (DOGS)

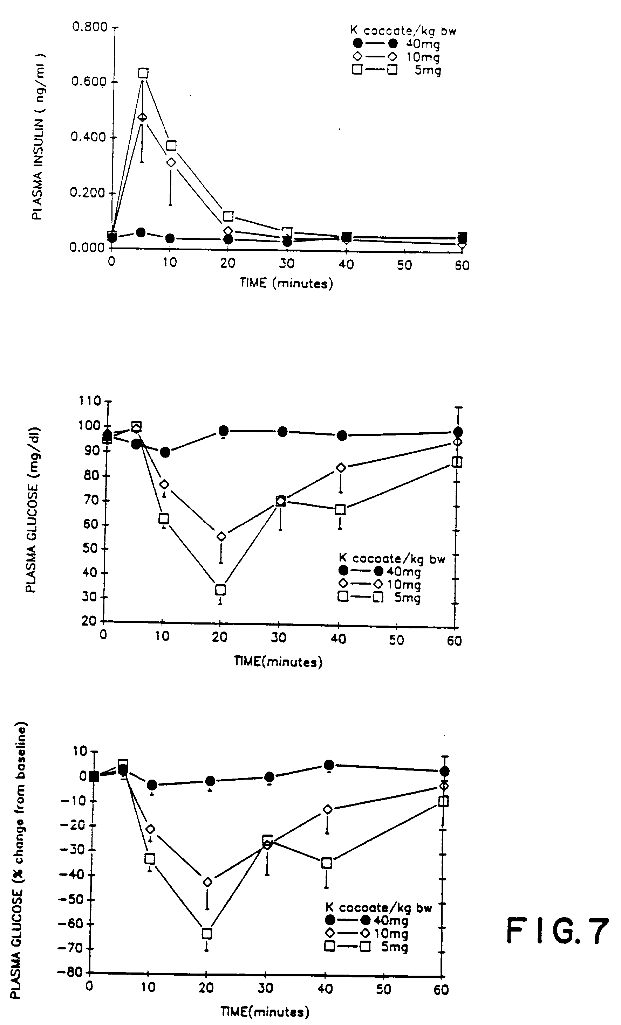

Mixtures of potassium cocoate and pork insulin (Lilly's zinc-insulin crystals pork purified) were infused intraduodenally into five fasting, awake beagle dogs chronically fitted with a modified Thomas cannula implanted in the distal duodenum. Each dog weighed about 10-15 kilograms. In three separate experiments, the dosage amount of the absorption enhancer was approximately either 40, 10 or 5 milligrams per kilogram of body weight and of the insulin was approximately 12 units per kilogram of body weight. Blood samples for monitoring glucose and insulin levels were obtained pre-dose and 5, 10, 20, 30, 40 and 60 minutes post-dose. The results are shown in Figure 7 for both plasma glucose and plasma insulin concentrations, with data points representing mean ± S.E.M.

Intraduodenal administration of the absorption enhancer-insulin mixture resulted in a significant decrease in plasma glucose (-42 and -63%) by 20 minutes after infusion in the 10 mg and 5 mg potassium cocoate groups, respectively, while no significant change occurred in the 40 mg group. These studies show that the absorption enhancer does increase the intestinal absorption of insulin in conscious, unrestrained dogs to produce a significant, reproducible augmentation of plasma insulin. However, the absorption of insulin is dependent upon the concentration of the absorption enhancer.

The administration of absorption enhanced insulin-containing dosage forms according to this invention and the resulting effect on plasma glucose and plasma insulin concentrations are shown in the following animal studies.

## Buccal Administration

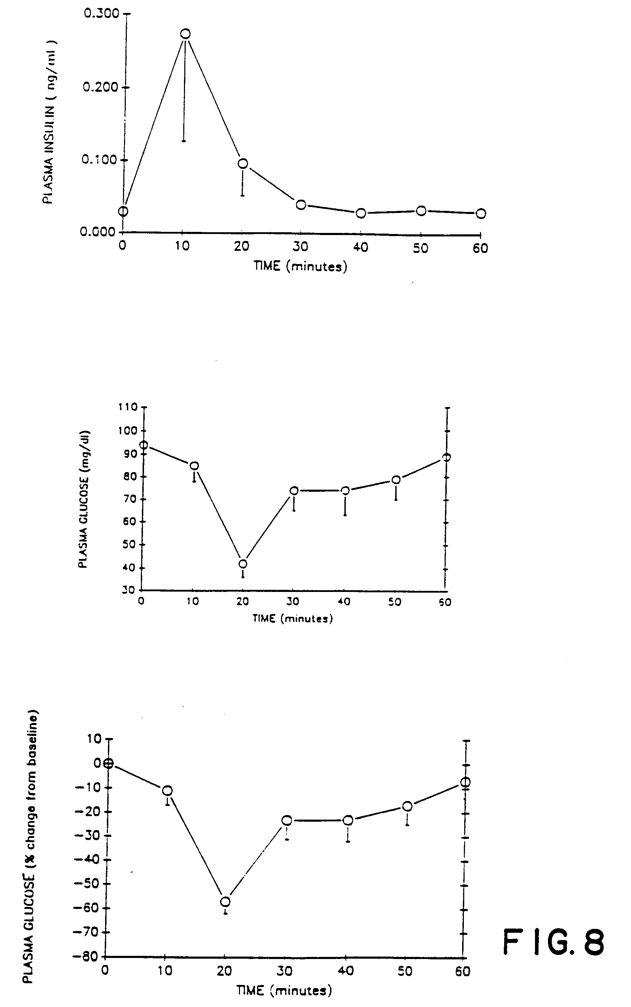

A mixture of potassium cocoate and insulin was placed in the cheek pouch of each of five beagle dogs (weighing approximately 12 kg each). The formulation was prepared by mixing 100 mg of potassium cocoate with 120 units of crystalline insulin (Sigma Chemical Company) dissolved in water, resulting in a final volume of 0.3 ml, which was administered to each dog. The dosage amount of the absorption enhancer was 10 mg per kilogram of body weight and of the insulin was 12 units per kilogram of body weight. The results are shown in Figure 8 for plasma glucose and plasma insulin concentrations, with data points representing mean $\pm$ S.E.M.

Buccal administration of the absorption enhancer-insulin mixture resulted in a significant decrease in plasma glucose (57%) by 20 minutes after dosing. Plasma insulin concentration increased to a 9-fold peak after 10 minutes and remained elevated for 20 minutes after dosing. This study shows that significant absorption of insulin into the bloodstream occurs following buccal administration of the absorption enhancer-insulin mixture. Furthermore, the absorption of insulin was similar following both intraduodenal and buccal administration of the same absorption enhancer-insulin mixture.

## Administration of Insulin and Absorption Enhancers as Buccal Films

Buccal films were prepared with varying amounts of potassium cocoate, hydroxypropyl cellulose and crystalline insulin. Each film was placed in the cheek pouch of each of twelve beagle dogs (weighing approximately 10 kg each). The dosage amounts of potassium cocoate, the absorption enhancer, ranged from about 1-20 mg per kilogram of body weight and the dosage amounts of insulin concentration ranged from about 6-12 units per kilogram of body weight.

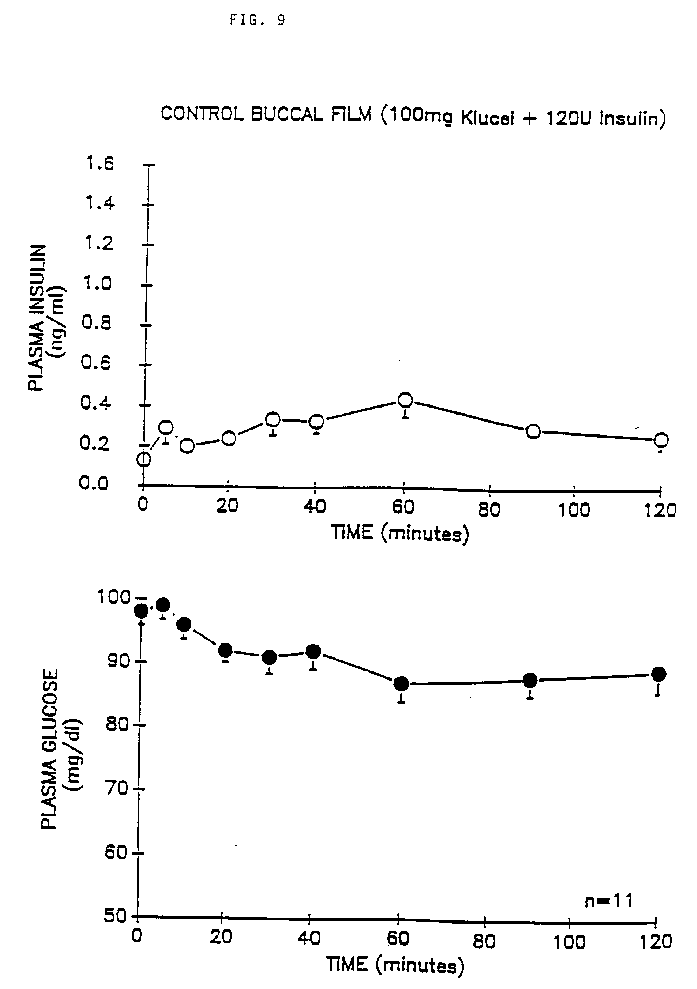

A control buccal film was prepared having a formulation of 100 mg hydroxypropyl cellulose and 120 units of crystalline insulin (Lilly), with no absorption enhancer. The film was placed in the cheek pouch of eleven beagle dogs. Plasma insulin concentration was elevated slightly and plasma glucose was observed to decrease slightly and then remain relatively constant for over two hours as shown in Figure 9.

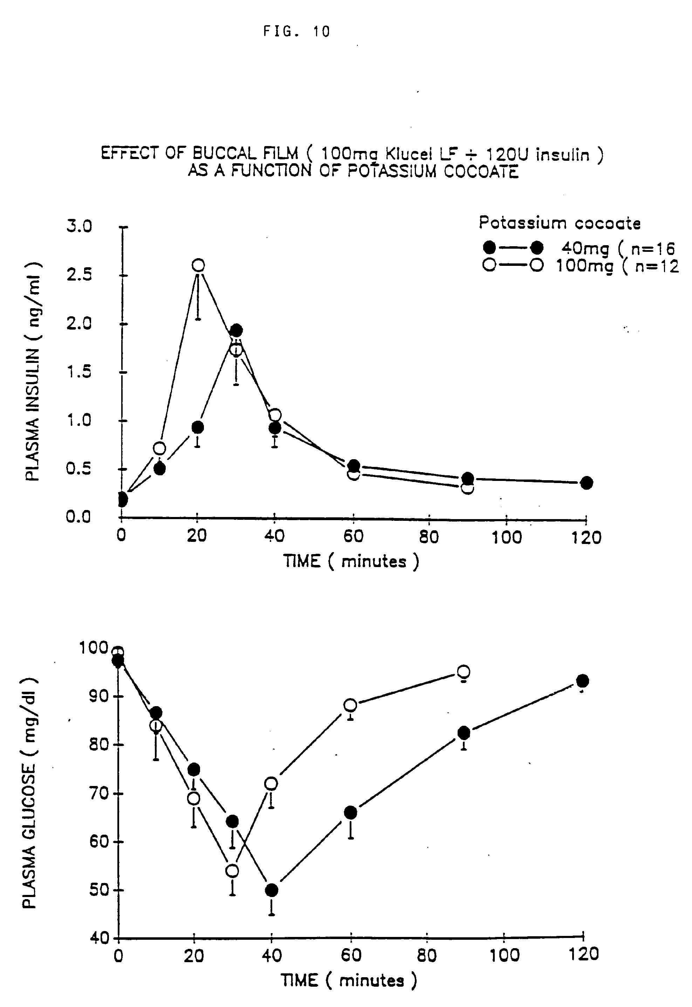

Buccal films having 60 units of crystalline insulin (Lilly) mixed with 20 or 40 mg of potassium cocoate and 50 or 100 mg of hydroxypropyl cellulose (Klucel LF®) were prepared. The resulting films were cut into circular forms of about 10 cm² surface area and placed in the buccal pouch of dogs. As shown in Figure 10 (left panel), plasma glucose was unchanged with the formulation of 60 units insulin, 20 mg potassium cocoate and 100 mg of Klucel LF® but when the film contained half as much Klucel LF®, plasma glucose decreased 30% by 40 minutes after dosing. In films containing 60 units of insulin mixed with mg of potassium cocoate and 100 mg of Klucel LF®, plasma glucose concentration decreased about 15% by 40 minutes after dosing but when the film contained half as much Klucel LF®, plasma glucose decreased 44% by 30 minutes after dosing (See Figure 10 right panel).

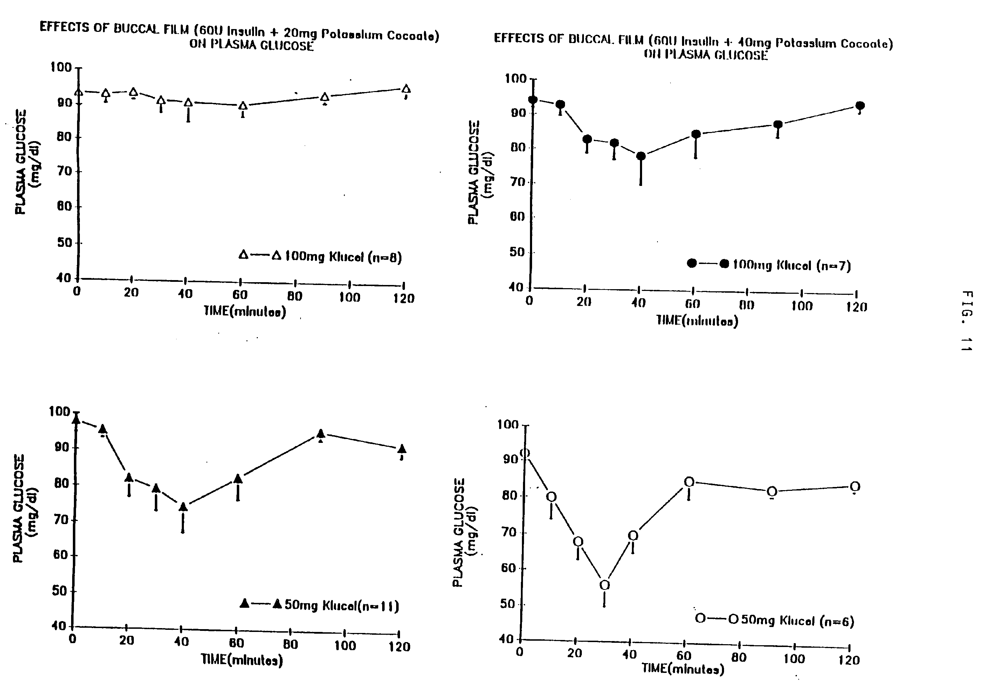

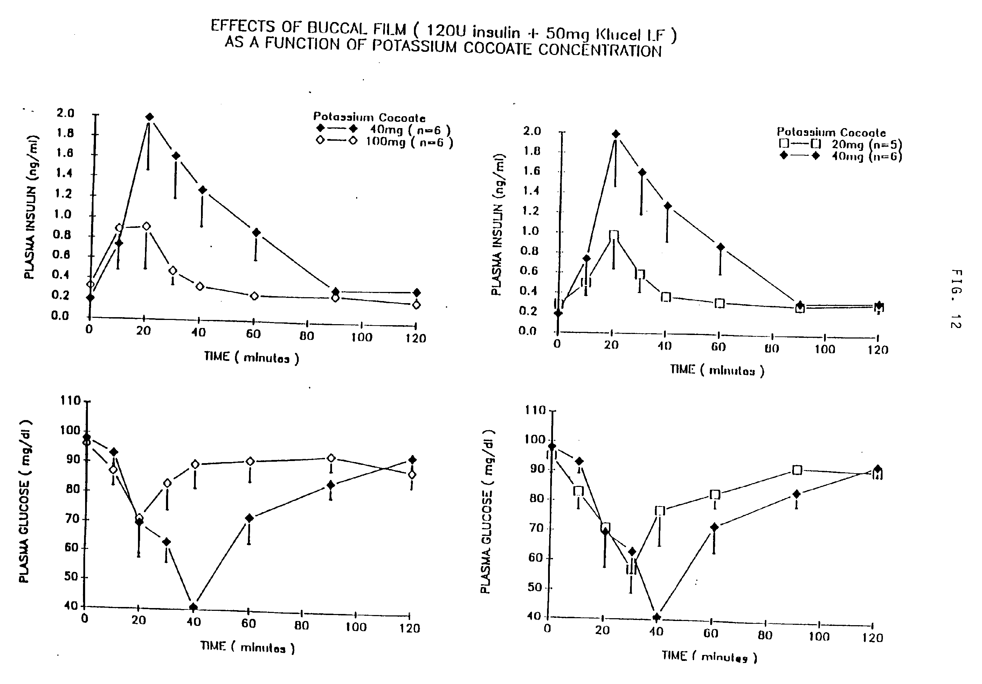

Formulations having 120 units of insulin in combination with 20, 40, or 100 mg of potassium cocoate and 50 or 100 mg of Klucel LF® were prepared and placed in the buccal cavity of normal dogs. Plasma insulin concentration was observed to increase significantly within 20 minutes after dosing and plasma glucose was observed to decrease significantly by 40 minutes after dosing as illustrated in Figures 11 and 12 .

A different time course of insulin absorption was observed with films containing 40 mg potassium cocoate compared to 100 mg potassium cocoate added to a fixed concentration of 120 units of insulin and 100 mg Klucel LF®. The plasma insulin peak occurred 30 minutes after dosing compared to 20 minutes with the 40 mg (n=16) and 100 mg (n=12) potassium cocoate formulations, respectively. These insulin responses resulted in a more prolonged plasma glucose decrease (up to 2 hours) with the formulation containing less potassium cocoate (Figure 11). The most significant effects were observed with buccal films having 40 mg of potassium cocoate mixed with 50 mg of Klucel LF® (see Figure 12).

This study shows that rapid, physiologically significant absorption of insulin into the bloodstream occurs following the administration of an insulin-absorption enhancer mixture using a buccal film in normal dogs.

The described dosage forms of this invention are, as mentioned and as demonstrated in the foregoing studies, useful to treat hyperglycemia. A preferred embodiment is the buccal film which is useful in treating hyperglycemia and particularly useful in treating post-prandial hyperglycemia. Since rapid insulin absorption into the blood has been observed with the buccal films, this dosage form may replace the meal associated insulin administration of the art with a more rapid delivery system and eliminate a major clinical problem associated with the high rise of glucose in the blood after meals. The buccal film and the other dosage forms described above, pertaining to a method of treatment, constitutes another aspect of this invention.

In the practice of the invention, the dose of the described insulin composition to be administered and the frequency of administration will depend on the potency and duration of activity of the insulin and the degree of absorption conferred by the absorption enhancer, as well as the severity of the condition, presence of neutralizing antibodies, degree of insulin resistance, duration of diabetes, age of the subject being treated and other factors known to the skilled practitioner. Doses of insulin contemplated for use in the practice of the invention are typical for insulin treatment as delivered to the bloodstream by injectable forms of insulin, for example, from 25 to 500 units per day, depending on specific needs, either as a single dose or in divided doses per day.

BRIEF DESCRIPTION OF THE DRAWINGS

Fig. 1- This drawing graphs blood glucose versus time following the intraduodenal administration of a single dose of an insulin-absorption enhancer combination in rats.

Fig. 2 - This drawing graphs plasma glucose and plasma insulin responses versus time as a function of absorption enhancer concentration at a constant dose of insulin (20 units), following intraduodenal administration using rats as the subjects.

Fig. 3 - This drawing graphs plasma glucose and plasma insulin responses versus time as a function of insulin concentration at a constant amount of absorption enhancer (9.2 mg), following intraduodenal administration using rats as the subjects.

Fig. 4 - This drawing graphs plasma glucose and plasma insulin responses versus time as a function of insulin concentration at a constant amount of absorption enhancer (4.6 mg), following intraduodenal administration using rats as the subjects.

Fig. 5 - This drawing graphs plasma glucose and plasma insulin responses versus time, using a constant dose of insulin and absorption enhancer, with intraduodenal administration in a multiple dose study in rats.

Fig. 6- This drawing graphs blood glucose versus time following intraduodenal administration of a single dose of an insulin-absorption enhancer mixture in a diabetic rat.

Fig. 7 - This drawing graphs plasma glucose and plasma insulin responses versus time following the intraduodenal administration of a single dose of three insulin-absorption enhancer mixtures to dogs.

Fig. 8 - This drawing graphs plasma glucose and plasma insulin responses versus time following the buccal administration of an insulin-absorption enhancer mixture to dogs.

Fig. 9 - This drawing graphs plasma glucose and plasma insulin responses versus time following the administration of a control buccal film (hydroxypropyl cellulose (100 mg) and insulin (120 units)) containing no absorption enhancer to dogs.

Fig. 10 - This drawing graphs plasma glucose responses versus time following the administration of buccal films containing varying mixtures of absorption enhancer (i.e. 20 mg and 40 mg) and hydroxypropyl cellulose (i.e. 50 mg and 100 mg) with a fixed amount of insulin (60 units) to dogs.

Fig. 11 - This drawing graphs plasma insulin and glucose responses versus time following the administration of buccal films as a function of absorption enhancer concentration at a fixed amount of insulin (120 units) and hydroxypropyl cellulose (100 mg) to dogs.

Fig. 12 - This drawing graphs plasma insulin and glucose responses versus time following the administration of buccal films as a function of absorption enhancer concentration at a fixed amount of insulin (120 units) and hydroxypropyl cellulose (50 mg) to dogs.

**Claims**

**Claims for the following Contracting States : AT, BE, CH, DE, DK, FR, GB, IT, LI, LU, NL, SE**

1.  A non-parenteral pharmaceutical composition comprising
    (a) a blood sugar-lowering amount of insulin,
    (b) an amount of an alkali metal cocoate sufficient to enhance the absorption of the insulin into the body, and
    (c) optionally, a pharmaceutically acceptable carrier.

2.  A composition according to claim 1, in which the cocoate comprises a mixture of two or more $C_8$-$C_{18}$ fatty acids or esters of such acids with glycerol.

3.  A composition according to claim 2, in which the fatty acids comprise caprylic, capric, lauric, myristic, palmitic and stearic acids.

4. A composition according to claim 2, in which the alkali metal of the cocoate is potassium.

5. A composition according to claim 1, in which the weight ratio of component (b) to component (a) is in the range from 500:1 to 1:1.

6. A composition according to claim 5, in which the weight ratio of component (b) to component (a) is in the range from 60:1 to 20:1.

7. A composition according to claim 1, which contains from 20 to 500 units of insulin per dosage unit.

**Claims for the following Contracting State : ES**

1. Process for the manufacture of a non-parenteral pharmaceutical composition which process comprises compounding
   (a) a blood sugar-lowering amount of insulin,
   (b) an amount of an alkali metal cocoate sufficient to enhance the absorption of the insulin into the body, and
   (c) optionally, a pharmaceutically acceptable carrier and bringing the mixture into non-parenteral pharmaceutical dosage units.

2. A process according to claim 1, in which the cocoate comprises a mixture of two or more $C_8$-$C_{18}$ fatty acids or esters of such acids with glycerol.

3. A process according to claim 2, in which the fatty acids comprise caprylic, capric, lauric, myristic, palmitic and stearic acids.

4. A process according to claim 2, in which the alkali metal of the cocoate is potassium.

5. A process according to claim 1, in which the weight ratio of component (b) to component (a) is in the range from 500:1 to 1:1.

6. A process according to claim 5, in which the weight ratio of component (b) to component (a) is in the range from 60:1 to 20:1.

7. A process according to claim 1, in which a dosage unit contains from 20 to 500 units of insulin.

**Patentansprüche**

**Patentansprüche für folgende Vertragsstaaten : AT, BE, CH, DE, DK, FR, GB, IT, LI, LU, NL, SE**

1. Eine nicht-parenterale pharmazeutische Komposition, umfassend
   (a) eine Blutzucker-senkende Menge Insulin,
   (b) eine zur Verstärkung der Insulinresorption im Körper hinreichende Menge eines Alkalicocoats, und
   (c) gewünschtenfalls einen pharmazeutisch akzeptablen Träger.

2. Eine Komposition gemäss Anspruch 1, in der das Cocoat ein Gemisch von zwei oder mehr $C_{8-18}$ - Fettsäuren oder Ester solcher Säuren mit Glyzerin umfasst.

3. Eine Komposition gemäss Anspruch 2, in der die Fettsäuren Capryl-, Caprin-, Lauryl-, Myristin-, Palmitin- und Stearinsäre umfassen.

4. Eine Komposition gemäss Anspruch 2, in der das Alkalimetall des Cocoats Kalium ist.

5. Eine Komposition gemäss Anspruch 1, in der das Gewichtsverhältnis von Komponente (b) zu Komponente (a) im Bereich von 500:1 bis 1:1 liegt.

6. Eine Komposition gemäss Anspruch 5, in der das Gewichtsverhältnis von Komponente (b) zu Komponente (a) im Bereich von 60:1 bis 20:1 liegt.

13

7.   Eine Komposition gemäss Anspruch 1, die 20 bis 500 Einheiten Insulin pro Dosiseinheit enthält.

**Patentansprüche für folgenden Vertragsstaat : ES**

1.   Verfahren zur Herstellung einer nicht-parenteralen pharmazeutischen Komposition, wobei
        (a) eine Blutzucker-senkende Menge Insulin,
        (b) eine zur Verstärkung der Insulinresorption im Körper hinreichende Menge eines Alkalicocoats, und
        (c) gewünschtenfalls ein pharmazeutisch akzeptabler Träger miteinander vermischt und das Gemisch
        in eine nicht-parenterale pharmazeutische Dosierungseinheit gebracht wird.

2.   Verfahren gemäss Anspruch 1, wobei das Cocoat ein Gemisch von zwei oder mehr $C_{8-18}$- Fettsäuren oder Ester solcher Säuren mit Glyzerin umfasst.

3.   Verfahren gemäss Anspruch 2, wobei die Fettsäuren Capryl-, Caprin-, Lauryl-, Myristin-, Palmitin- und Stearinsäre umfassen.

4.   Verfahren gemäss Anspruch 2, wobei das Alkalimetall des Cocoats Kalium ist.

5.   Verfahren gemäss Anspruch 1, wobei das Gewichtsverhältnis von Komponente (b) zu Komponente (a) im Bereich von 500:1 bis 1:1 liegt.

6.   Verfahren gemäss Anspruch 5, wobei das Gewichtsverhältnis von Komponente (b) zu Komponente (a) im Bereich von 60:1 bis 20:1 liegt.

7.   Verfahren gemäss Anspruch 1, wobei eine Dosiseinheit 20 bis 500 Einheiten Insulin enthält.


**Revendications**

**Revendications pour les Etats contractants suivants : AT, BE, CH, DE, DK, FR, GB, IT, LI, LU, NL, SE**

1.   Composition pharmaceutique non parentérale, comprenant
        (a) une quantité d'insuline abaissant la glycémie,
        (b) une quantité d'un composé à base de coprah d'un métal alcalin suffisante pour activer l'absorption de l'insuline dans l'organisme, et
        (c) le cas échéant, un véhicule pharmaceutiquement acceptable.

2.   Composition selon la revendication 1, dans laquelle le composé à base de coprah comprend un mélange de deux ou plusieurs acides gras en $C_8$-$C_{18}$ ou d'esters de ces acides avec du glycérol.

3.   Composition selon la revendication 2, dans laquelle les acides gras comprennent les acides caprylique, caprique, laurique, myristique, palmitique et stéarique.

4.   Composition selon la revendication 2, dans laquelle le métal alcalin du composé à base de coprah est le potassium.

5.   Composition selon la revendication 1, dans laquelle le rapport pondéral du constituant (b) au constituant (a) est dans la gamme de 500 : 1 à 1 : 1.

6.   Composition selon la revendication 5, dans laquelle le rapport pondéral du constituant (b) au constituant (a) est dans la gamme de 60 : 1 à 20 : 1.

7.   Composition selon la revendication 1, qui contient de 20 à 500 unités d'insuline par unité posologique.

**Revendications pour l'Etat contractant suivant : ES**

1.   Procédé de fabrication d'une composition pharmaceutique non parentérale, ce procédé comprenant le mélange
        (a) d'une quantité d'insuline abaissant la glycémie,

(b) d'une quantité d'un composé à base de coprah d'un métal alcalin suffisante pour activer l'absorption de l'insuline dans l'organisme, et

(c) le cas échéant, d'un véhicule pharmaceutiquement acceptable, et la préparation d'unités posologiques pharmaceutiques non parentérales à partir du mélange.

2. Procédé selon la revendication 1, dans lequel le composé à base de coprah comprend un mélange de deux ou plusieurs acides gras en $C_8$-$C_{18}$ ou d'esters de ces acides avec du glycérol.

3. Procédé selon la revendication 2, dans lequel les acides gras comprennent les acides caprylique, caprique, laurique, myristique, palmitique et stéarique.

4. Procédé selon la revendication 2, dans lequel le métal alcalin du composé à base de coprah est le potassium.

5. Procédé selon la revendication 1, dans lequel le rapport pondéral du constituant (b) au constituant (a) est dans la gamme de 500 : 1 à 1 : 1.

6. Procédé selon la revendication 5, dans lequel le rapport pondéral du constituant (b) au constituant (a) est dans la gamme de 60 : 1 à 20 : 1.

7. Procédé selon la revendication 1, dans lequel une unité posologique contient de 20 à 500 unités d'insuline.

FIG.1

EP 0 414 080 B1

FIG. 2

FIG. 3

F I G. 4

19

FIG. 5

20U insulin + 8.3 mg potassium cocoate

F I G. 6

EP 0 414 080 B1

FIG.7

22

F IG. 8

FIG. 9

CONTROL BUCCAL FILM (100mg Klucel + 120U Insulin)

24

FIG. 10

EFFECT OF BUCCAL FILM ( 100mg Klucel LF ÷ 120U insulin )
AS A FUNCTION OF POTASSIUM COCOATE

FIG. 11

EFFECTS OF BUCCAL FILM (60U Insulin + 40mg Potassium Cocoate) ON PLASMA GLUCOSE

PLASMA GLUCOSE (mg/dl)

TIME(minutes)

●——● 100mg Klucel (n=7)

EFFECTS OF BUCCAL FILM (60U Insulin + 20mg Potassium Cocoate) ON PLASMA GLUCOSE

PLASMA GLUCOSE (mg/dl)

TIME(minutes)

△——△ 100mg Klucel (n=8)

PLASMA GLUCOSE (mg/dl)

TIME(minutes)

○——○ 50mg Klucel (n=6)

PLASMA GLUCOSE (mg/dl)

TIME(minutes)

▲——▲ 50mg Klucel (n=11)

26

FIG. 12

EFFECTS OF BUCCAL FILM ( 120U insulin + 50mg Klucel LF )
AS A FUNCTION OF POTASSIUM COCOATE CONCENTRATION